# EUROPEAN PATENT SPECIFICATION

(11) **EP 3 434 287 B1**
(45) Date of publication and mention of the grant of the patent: **27.01.2021**
(21) Application number: 17425081.1
(22) Date of filing: 25.07.2017
(51) Int. Cl.: A61K 47/54, C07K 5/11, C07K 7/06, A61P 31/04, A61P 31/10

(54) **SHORT AND ULTRA-SHORT ANTIMICROBIAL LIPOPEPTIDES AND USE THEREOF**
KURZE UND ULTRAKURZE ANTIMIKROBIELLE LIPOPEPTIDE UND VERWENDUNG DAVON
LIPOPEPTIDES ANTIMICROBIEN ET COURTS ULTRA-COURTE ET SON UTILISATION

(43) Date of publication of application: 30.01.2019
(73) Proprietor: Arta Peptidion S.r.l.s., 43126 Parma (IT)
(72) Inventor: Romani, Antonello, 43121 Parma (IT); Allodi, Tatiana, 43015 Noceto (IT); Benincasa, Monica, 34128 Trieste (IT); Scocchi, Marco, 34074 Monfalcone (IT)
(74) Representative: Petraz, Gilberto Luigi

(56) References cited:
- WO-A1-2010/109284
- WO-A2-2008/093058
- CN-A- 104 072 579
- FANG YUXIN ET AL: "Tuning the antimicrobial pharmacophore to enable discovery of short lipopeptides with multiple modes of action", EUROPEAN JOURNAL OF MEDICINAL CHEMISTRY, EDITIONS SCIENTIFIQUE ELSEVIER, PARIS, FR, vol. 83, 4 June 2014 (2014-06-04), pages 36-44, XP029010253, ISSN: 0223-5234, DOI: 10.1016/J.EJMECH.2014.06.003
- WAKABAYASHI H ET AL: "N-Acylated and D Enantiomer Derivatives of a Nonamer Core Peptide of Lactoferricin B Showing Improved Antimicrobial Activity", ANTIMICROBIAL AGENTS AND CHEMOTHERAPY, AMERICAN SOCIETY FOR MICROBIOLOGY, vol. 43, no. 5, 1 May 1999 (1999-05-01), pages 1267-1269, XP002212843, ISSN: 0066-4804
- Y. BROTMAN ET AL: "Synthetic Ultrashort Cationic Lipopeptides Induce Systemic Plant Defense Responses against Bacterial and Fungal Pathogens", APPLIED AND ENVIRONMENTAL MICROBIOLOGY, vol. 75, no. 16, 19 June 2009 (2009-06-19) , pages 5373-5379, XP55439667, ISSN: 0099-2240, DOI: 10.1128/AEM.00724-09
- HASSAN MAHMOOD JINDAL ET AL: "Antimicrobial Activity of Novel Synthetic Peptides Derived from Indolicidin and Ranalexin against Streptococcus pneumoniae", PLOS ONE, vol. 10, no. 6, 5 June 2015 (2015-06-05), page e0128532, XP055458392, DOI: 10.1371/journal.pone.0128532
- BRANDENBURG K ET AL: "Effective antimicrobial and anti-endotoxin activity of cationic peptides based on lactoferricin: A biophysical and microbiological study", ANTI-INFECTIVE AGENTS IN MEDICINAL CHEMISTRY, BENTHAM SCIENCE PUBLISHERS, HILVERSUM, NL, vol. 9, no. 1, 31 December 2009 (2009-12-31), pages 9-22, XP009504030, ISSN: 1871-5214, DOI: 10.2174/187152110790886736

### SEQUENCE LISTING

<110> ARTA PEPTIDION S.R.L.S.
<120> SHORT AND ULTRA-SHORT ANTIMICROBIAL LIPOPEPTIDES AND USE THEREOF
<130> G5-0942
<160> 20
<170> BiSSAP 1.3.6
<210> 1
   <211> 4
   <212> PRT
   <213> Homo sapiens
<220>
   <223> >1
<400> 1
   Arg Ala Trp Arg
   1
<210> 2
   <211> 4
   <212> PRT
   <213> Homo sapiens
<220>
   <223> >2
<400> 2
   Arg Cys Trp Arg
   1
<210> 3
   <211> 4
   <212> PRT
   <213> Homo sapiens
<220>
   <223> >3
<400> 3
   Arg Phe Trp Arg
   1
<210> 4
   <211> 4
   <212> PRT
   <213> Homo sapiens
<220>
   <223> >4
<400> 4
   Arg Gly Trp Arg
   1
<210> 5
   <211> 4
   <212> PRT
   <213> Homo sapiens
<220>
   <223> >5
<400> 5
   Arg His Trp Arg
   1
<210> 6
   <211> 4
   <212> PRT
   <213> Homo sapiens
<220>
   <223> >6
<400> 6
   Arg Ile Trp Arg
   1
<210> 7
   <211> 4
   <212> PRT
   <213> Homo sapiens
<220>
   <223> >7
<400> 7
   Arg Leu Trp Arg
   1
<210> 8
   <211> 4
   <212> PRT
   <213> Homo sapiens
<220>
   <223> >8
<400> 8
   Arg Met Trp Arg
   1
<210> 9
   <211> 4
   <212> PRT
   <213> Homo sapiens
<220>
   <223> >9
<400> 9
   Arg Arg Trp Arg
   1
<210> 10
   <211> 4
   <212> PRT
   <213> Homo sapiens
<220>
   <223> >10
<400> 10
   Arg Ser Trp Arg
   1
<210> 11
   <211> 4
   <212> PRT
   <213> Homo sapiens
<220>
   <223> >11
<400> 11
   Arg Val Trp Arg
   1
<210> 12
   <211> 4
   <212> PRT
   <213> Homo sapiens
<220>
   <223> >12
<400> 12
   Arg Trp Trp Arg
   1
<210> 13
   <211> 4
   <212> PRT
   <213> Homo sapiens
<220>
   <223> >13
<400> 13
   Phe Trp Arg Arg
   1
<210> 14
   <211> 4
   <212> PRT
   <213> Homo sapiens
<220>
   <223> >14
<400> 14
   Arg Arg Phe Trp
   1
<210> 15
   <211> 4
   <212> PRT
   <213> Homo sapiens
<220>
   <223> >15
<400> 15
   Phe Arg Arg Trp
   1
<210> 16
   <211> 6
   <212> PRT
   <213> Homo sapiens
<220>
   <223> >16
<400> 16
   Arg Arg Phe Trp Arg Arg
   1 5
<210> 17
   <211> 4
   <212> PRT
   <213> Homo sapiens
<220>
   <223> >17
<400> 17
   Arg Arg Ile Trp
   1
<210> 18
   <211> 4
   <212> PRT
   <213> Homo sapiens
<220>
   <223> >18
<400> 18
   Ile Trp Arg Arg
   1
<210> 19
   <211> 4
   <212> PRT
   <213> Homo sapiens
<220>
   <223> >19
<400> 19
   Trp Ile Arg Arg
   1
<210> 20
   <211> 6
   <212> PRT
   <213> Homo sapiens
<220>
   <223> >20
<400> 20
   Arg Arg Ile Trp Arg Arg
   1               5

## Description

### FIELD OF INVENTION

Embodiments of the present disclosure relate to lipopeptides having biological and therapeutic activity against intermediate-, multi- and extensively-resistant bacteria. This invention relates to short and ultra-short lipidated peptides with general formula FA-A-B-A'-NH2 that exhibit high or very high antimicrobial activity. The lipopeptides of this invention provide enhanced anti-microbial and anti-fungal activity over the inactive or weakly inactive base tetrapeptide or hexapeptides.

### STATE OF THE ART

Treatment of bacterial infections with antibiotics represents one of the pillars of human medicine. Development and employment of antibiotics, starting from the second half the XX century, have revolutionized the approach to treatment and prevention of infectious diseases and infections considered incurable in the past.

However, despite resources and energies invested to increase the knowledge of the resistance mechanisms, and in the research of ever more efficient molecules, now, the development of drug-resistance to antibiotics is faster than the discovery of new classes of molecules.

Today this problem has become a veritable public health priority on a global scale, also due to the appearance of pathogens simultaneously resistant to several antibiotics (multidrug resistance, or heterogeneous resistance), *de facto* reducing the possibility of an efficient treatment (WHO Global Strategy for Containment of Antimicrobial Resistance. Geneva, World Health Organization, 2001, WHO/CDS/CSR/DRS/2001.2). Heterogeneous antibiotic-resistance often involves health structures. These multi-resistant microorganisms deriving from hospital acquired infections can be transmitted in a community in various ways among which: ventilation and aeration systems, water flow, treatment of tissues and laboratory samples, inadequate hygiene of personnel and environment, surgical procedures and invasive devices (Eggimann, Clin Microbiol Infect 2001;7:91; Pittet, The Lancet 2000;356: 1307; Hugonnet, Clin Microbiol Infect 2000;6:350; Pittet, Swiss-NOSO 2001 ;8:25).

Among the major problems, methicillin-resistance in *S. aureus* should be mentioned ("Celbenin"-resistant staphylococci. Br Med J, i: 124-25, 1961), which prevalence in Italy has reached a constant incidence around 40%, and is among the highest in Europe (Antimicrobial resistance surveillance in Europe 2009. Annual Report of the European Antimicrobial Resistance Surveillance Network (EARS-Net). Stockholm, European Centre for Disease Prevention and Control (ECDC), 2010). Penicillin-resistance in S. pneumoniae reaches a frequency higher than 20% in some European countries and in the United States, and it even exceeds 50% in the Far East. Recently, high macrolide-resistance was observed in this microorganism, with values reaching 30%, with an increasing trend. In the genus Enterococcus, the frequency of vancomycin-resistance has progressively increased all over the world, in the last years (around 20% in the United States) (in Centers for Disease Control and Prevention. 2002. Morb Mortal Weekly Rep, July 5, 51 (26): 565-567). In Italy, from a study carried out in 2007, a frequency of vancomycin-resistance of about 2.5% in E. faecalis and of about 20% in the less frequent *E. faecium* was found (European Antimicrobial Resistance Surveillance System, EARSS, 2007).

In Gram-negative microorganisms, a very heterogeneous distribution of resistances is observed, with an increasing trend, related to fluoroquinolone-, aminopenicillin- and aminoglicoside-resistance in E. coli and K. pneumoniae/oxytoca, and carbapenem-resistance in P. aeruginosa ("Antibiotic-resistance surveillance and use of systemic antibiotics in Emilia-Romagna" Dossier 173/ 2009, Agenzia sanitaria e sociale regionale dell'Emilia-Romagna). Recently, the increased incidence of wide range beta-lactamase-producing Gram-negative bacteria has awakened remarkable interest for the epidemiological/ microbiological consequences and for the importance in therapy (from European Centre for Disease Prevention and Control (ECDC) and European Medicines Agency (EMEA). ECDC/ EMEA Joint Technical Report - The bacterial challenge: time to react. Stockholm, 2009).

Just like antibiotics cure bacterial infections, antifungal medications save lives by curing dangerous fungal infections. And just like some bacterial infections are resistant to antibiotics, some fungi no longer respond to the antifungal medications that are designed to cure them. This emerging phenomenon is known as antifungal resistance, and it's primarily a concern for invasive infections with the fungus *Candida.* Although antibiotic-resistant bacterial infections are a widely-recognized public health threat, less is known about the effects of antifungal resistance and the burden of drug-resistant fungal infections. Invasive fungal infections cause substantial morbidity and mortality and are a costly, common problem in healthcare settings. The fungus *Candida* is the most common cause of healthcare-associated bloodstream infections in the United States. (Magill SS, Edwards JR, Bamberg W, et al. Multistate point-prevalence survey of health care-associated infections. The New England journal of medicine 2014; 370:1198-208). Each case of *Candida* bloodstream infection is estimated to result in an additional 3 to 13 days of hospitalization and $6,000 to $29,000 in healthcare costs *(*Morgan J, Meltzer MI, Plikaytis BD, et al. Excess mortality, hospital stay, and cost due to candidemia: a case-control study using data from population-based candidemia surveillance. Infection control and hospital epidemiology 2005; 26:540-7*).*

What's also concerning is that some types of *Candida* are becoming increasingly resistant to first-line and second-line antifungal medications, namely, fluconazole and echinocandins (anidulafungin, caspofungin, and micafungin) (Vallabhaneni S, Cleveland A, Farley M, et al. Epidemiology and Risk Factors for Echinocandin Nonsusceptible Candida glabrata Bloodstream Infections: Data from a Large Multisite Population-Based Candidemia Surveillance Program, 2008-2014. Open Forum Infect Diseases 2015; 2:163*).* Approximately 7% of all *Candida* bloodstream isolates tested at CDC are resistant to fluconazole, most of which are *Candida glabrata (*Lockhart SR, Iqbal N, Cleveland AA, et al. Species identification and antifungal susceptibility testing of Candida bloodstream isolates from population-based surveillance studies in two U.S. cities from 2008 to 2011. Journal of clinical microbiology 2012; 50:3435-42*).* CDC's surveillance data indicate that the proportion of *Candida* isolates that are resistant to fluconazole has remained constant over the past twenty years. Echinocandin resistance, however, appears to be on the rise, especially among *Candida glabrata.* CDC's surveillance data indicate that up to 8% of *Candida glabrata* isolates in 2014 may not be susceptible to echinocandins; this proportion nearly doubled from 4% in 2008. This is especially concerning as echinocandins are the mainstay of treatment for *Candida glabrata,* which already has high levels of resistance to fluconazole (Alexander BD, Johnson MD, Pfeiffer CD, et al. Increasing echinocandin resistance in Candida glabrata: clinical failure correlates with presence of FKS mutations and elevated minimum inhibitory concentrations. Clinical infectious diseases 2013; 56:1724-32*).*

Antimicrobial peptides represent a further route for the innate response to microbial infections (Hartmann M., et al. 2010, Antimicrob. Agents Chemother. 54:3132-3142). They have double importance, in that they protect about 80% of the animal species and almost all the plants, and play a key role also in immunity of superior animals, providing a sort of first line of defence that stimulates and actively cooperates with the adaptive immune responses (Tossi A and Sandri L., Curr. Pharm. Design, 2002 8: 743-761). In superior animals, they represent the "effector" molecules of innate immunity (Boman HG., J Intern Med. 2003 254: 197-215).

Antimicrobial peptides show a wide spectrum of activity: they quickly kill bacterial cells and are active against various clinically relevant antibiotic-resistant strains (Hancock and Chappie, Antimicrob Agents Chemother, 1999 43: 1317-1323; Proc Natl Acad Sci USA. 2000 97:8856-886; Zasloff M., Nature, 2002 415:389-395).

It has been additionally demonstrated that, for bacteria, it is much more difficult to become resistant to proteins with antimicrobial activity, in that this would require a rearrangement or modification of the lipidic composition of their membrane, an uneconomic and "costly" process for all microbial species (Zasloff M., Nature, 2002 415:389-395).

Most antimicrobial peptides act by directly altering the membrane of target cells (Thevissen et al., Mol Plant-Microbe Interact. 2000, 13:54-61). Bacterial membranes are rich in anionic phospholipids, such as phosphatidylserine and phosphatidylglycerol: this determines an electrostatic interaction of the positively charged peptide with the membrane itself, that is the basis of the subsequent perturbation effect of the double layer.

In the case of Gram-negative bacteria, it has been seen that the peptide first interacts with the polyanionic lipopolysaccharide molecules of the external membrane, and then it is able to permeabilize it or to be captured inside. In the case of Gram-positive bacteria, instead, the peptide is probably attracted by teichoic and teichuronic acids and by other anionic groups found externally on the peptidoglycan layer. The different composition of the membranes, in fact, is the basis of the selectivity that some of these peptides have for bacterial cells.

Eukaryotic cell membranes are characterized by a high content of zwitterionic phospholipids, such as phosphatidylcholine, sphingomyelin, and phosphatidylethanolamine. Additionally, they are rich in cholesterol, absent in bacteria, which seems to inhibit the action of such peptides giving membranes a certain resistance (Zasloff M., Nature, 2002 415:389-395).

Another important selectivity factor is the value of the membrane potential: a more negative potential inside the cell, typical of bacterial cells (100-150 mV), eases the interaction of the peptide with the lipidic layer (Bechinger B. J Membr Biol. 1997 156: 197-21 1).

Additionally, it has been observed that many peptides with proven antibacterial *in vitro,* once tested in other culture media *in vitro,* or *in vivo,* lose such antibacterial activity or most of it; such phenomenon has been attributed to the inactivating action on peptides by high salt concentrations, such as those present in different compartments of the human body or in some culture media as well as the action of serum and tissues peptidases. Hence, it would be further useful to have groups or subgroups of peptides that, in addition to a useful basal antimicrobial activity, retained the same also in the presence of high salt concentrations along with protease and peptidedase resistance, so as to obtain a consistent antibacterial effect *in vivo* in all the physiological compartments.

Publication Fang Yuxin et al. "Tuning the antimicrobial pharmacophore to enable discovery of short lipopetides wih multiple modes of action", European Journal of Medicinal Chemistry, Editions Scientifique Elsevier, Paris, FR, vol. 83, 4 June 2014 discloses synthesized very short arginine, lysine and tryptophan containing lipopetides and the evaluation of their antimicrobial activity against a panel of pathogenic microorganisms including *Staphilococcus aureus, Escherichia coli* and *Candida albicans.*

Document CN 104072578 A discloses small molecule peptides with antibacterial and antimicrobial activity.

There is therefore a need to improve short and ultra-short antimicrobial lipopeptides and use thereof, which overcome at least one of the drawbacks in the art.

In particular, one purpose of the present disclosure is to provide cost effective short and ultra-short antimicrobial lipopeptides which can be used in topical and systemic treatment of conditions associated with intermediate drug resistant and drug resistant bacterial and fungal infection.

Various limitations and disadvantages of conventional solutions and technologies will become apparent to one of skill in the art after reviewing the remainder of the present application with reference to the drawings and description of the embodiments which follow, though it should be understood that this description of the related art section is not intended to serve as an admission that the described subject matter is prior art.

### SUMMARY OF INVENTION

In response to the drawbacks showed by antibiotics and antifungals currently on the market or available for the treatment of antibiotic- or antifungal-resistant infections, an object of the present invention is a series of short and ultra-short lipopeptides with antimicrobial and antifungal activity and wide spectrum of activity.

It is now disclosing that the conjugation of a lipophilic moiety, particularly a fatty acid, to an inactive or weakly active antimicrobial peptide unexpectedly endow the peptide with high antimicrobial activity.

Embodiments described herein refer to a lipopeptide having a sequence FA-A-B-A'-NH2, wherein:
- FA represents a fatty acid selected from caproic acid (hexanoic), caprylic acid (octanoic), capric acid (decanoic), lauric acid (dodecanoic), mystiric acid (tetradecanoic), pentadecanoic acid and palmitic acid (hexadecanoic);
- unit A and A' independently consist of 1, 2 or 3 amino acids selected from the group of basic amino acids or hydrophobic amino acids;
- unit B consist of 1, 2 or 3 amino acids, selected from the group (a) of hydrophobic amino acids and (b) the group of hydrogen-bond forming amino acids; wherein:
   (i) said hydrophobic amino acids are selected from: Ala, Phe, Ile, Leu, Pro, Tyr, Trp, Val, Met, Cys;
   (ii) said basic amino acids are selected from: Lys, His, Arg;
   (iii) said hydrogen bond-forming amino acids are selected from Asn, Gin, Ser, Thr;
- -NH2 represents C-terminal amidation.

In some embodiments, the substructure A-B-A' contains from 2 to 4 points of alternation between amino acids of group (a) and group (b), or vice versa.

In some embodiments, at least one-third of said aminoacids are selected from the group of proteinogenic (e.g. alanine, arginine, serine), non-proteinogenic (e.g. norvaline, ornithine) or non-standard (e.g. hydroxyproline, selenomethionine, 3-(1-naphthyl)-alanine) amino acids.

In some embodiments, at least one amino acid is selected from the group of D-amino acids.

In some embodiments, FA can be defined as Cm, wherein m is an integer selected from 8, 9, 10, 12, 14, 15, 16 such that Cm represents a fatty acid selected from: caproic acid (hexanoic, C6), caprylic acid (octanoic, C8), capric acid (decanoic, c10), lauric acid (dodecanoic, C12), mystiric acid (tetradecanoic, C14), pentadecanoic acid (C15) and palmitic acid (hexadecanoic, C16).

In some embodiments, FA can be defined as Cn, wherein n is an integer selected from 12, 14, 15, 16 such that Cn represents a fatty acid selected from: lauric acid (dodecanoic, C12), mystiric acid (tetradecanoic, C14), pentadecanoic acid (C15) and palmitic acid (hexadecanoic, C16).

According to the present invention, A-B-A' is a sequence selected from the group of the following sequences: SEQ ID No.: 1, SEQ ID No.: 2, SEQ ID No.: 3, SEQ ID No.: 4, SEQ ID No.: 5, SEQ ID No.: 6, SEQ ID No.: 7, SEQ ID No.: 8, SEQ ID No.: 9, SEQ ID No.: 10, SEQ ID No.: 11.

Other sequences described herein, which are not part of the present invention, are: SEQ ID No.: 12, SEQ ID No.: 13, SEQ ID No.: 14, SEQ ID No.: 15, SEQ ID No.: 16, SEQ ID No.: 17, SEQ ID No.: 18, SEQ ID No.: 19, SEQ ID No.: 20.6.

In some embodiments, n is 12, such that FA is Lauryl (C12).

According to embodiments, the present disclosure further relates to a composition comprising at least one lipopeptide according to claim 1 and a pharmaceutically acceptable carrier.

In some embodiments, said lipopeptide is present in a concentration ranging from about 1 µg/mL to about 12.5% (w/v).

In some embodiments, said composition is in the form of an aerosol, cosmetic preparation, spray, emulsion, liquid, lotion, cream, paste, ointment, powder or foam.

In some embodiments, the composition of the present disclosure can be useful for the prevention or treatment of skin infections caused by bacteria or fungi, ear infections caused by bacteria or fungi, bacterial or fungal vaginosis.

In some embodiments, the composition of the present disclosure can also be employed in the treatment of infections of vegetal organisms.

Further embodiments relate to a method for preventing or treating a microbial infection of the skin or mucosal tissue of a mammal, the method comprising administering to the infected area of said mammal a therapeutically effective amount of a composition according to the present disclosure.

In some embodiments, in the above method, said therapeutically effective amount of the composition comprises said polipeptide at a concentration ranging from about 1 µg/mL to about 12.5% (w/v).

According aspects, the present disclosure can provide a lipophilic conjugate comprising a peptide with length that can be of 3 to 15 amino acids, with a net positive charge greater than +1 and, at least, two positively charged amino acids and at least one hydrophobic amino acid, coupled to a fatty acid.

After conjugation to the fatty acid the said peptide has at least one activity selected from the group consisting of antibacterial, antifungal, antiviral and anticancer activity wherein the activity of said peptide after conjugation being significant higher than prior to conjugation. It should be noted that said lipopeptide with antibacterial and/or antifungal and/or anticancer activity, do not to imply that the activity affects all bacteria or fungi species nor all cancer cell types.

In a preferred embodiment, the net positive charge can be equal or greater than +2. Accordingly, to another one preferred embodiment of the invention, the lipopeptide consist of at least four amino acid residues up to ten.

According to currently more preferred embodiments, the lipopeptide consists of 4 to 9 amino acid residues. More specifically, lipopeptides show a sequence of FA-A-B-A'-NH2 type, where: units FA represent a fatty acid selected from the group consisting of saturated, unsaturated, monounsaturated, and polyunsaturated fatty acid; units A and A' consist of 1,2,3 amino acids independently selected from the group of basic amino acid or hydrophobic amino acids; units B consist of 1,2,3 amino acids selected from: (a) hydrophobic amino acids or (b) basic amino acids or amino acids forming hydrogen bonds; The substructure A-B-A' is characterized in that it contains 2 to 6 points of alternation between amino acids of group (a) and group (b), or vice versa. Accordingly, to the principles of the present invention, the peptide prior to conjugation to a fatty acid is either inactive or weakly active antibacterial and/or antifungal and/or antiviral.

Preferably, conjugation of a fatty acid to a peptide of the invention enhances at least one activity selected from antibacterial, antifungal, antiviral, and anticancer activity by at least 2-fold, more preferably by at least 10-fold, and most preferably by at least 20-fold. Such lipopeptides, eventually, show high solubility in aqueous solvents. The invention also relates to the use of said lipopeptides in the treatment of infections concerning different districts (for example, pulmonary, gastrointestinal, urinary), cutaneous infections and medical/surgical diseases complicated by bacterial or fungal superinfections.

The lipopeptides of the present disclosure can be readily synthesized, highly efficient, proteolytically stable, essentially salt-insensitive, with low, if any, hemolytic and/or cytotoxic activity towards eukaryotic cells.

The antibacterial spectrum of the antimicrobial lipopeptides of the present invention includes Gram-negative and Gram-positive bacteria, Fungi and Yeasts. Gram-negative microorganisms are, for example, *Escherichia coli* ATCC 25922, *Pseudomonas aeruginosa* ATCC 27853: in particular, MIC against *E. coli* range between 7.8µM (6.25 µg/ml) and 15.6µM (12.5 µg/ml) and for *P. aeruginosa* between 15.6 µM (12.5 µg/ml). and 80 µM (100 µg/ml) were observed. Of interest are the results obtained against Gram-positive bacteria, against S. aureus (ATCC 25923), with MIC comprised between 1.875 µM (1.5 µg/ml) and 7.8 µM (6.25 µg/ml). Equally interesting are the results obtained with strain of *S. mutans* (ATCC 35668) with a MIC of 7.8 µM (6.25 µg/ml), *Listeria Monocytogenes* (DSM 20600) with a MIC of 1.875 µM (1.5 µg/ml). Particularly interesting is the result obtained against a clinical strain of vancomycin-resistant Enterococcus faecalis, with a MIC of 7.8 µM (6.25 µg/ml). The lipopeptides of the present invention demonstrated to be highly active also against fungal and yeast species, as highlighted by the activity against *C*. *albicans* ATCC 90029 with a MIC of 1.875 µM (1.56 µg/ml). Furthemore, lipopeptides of the present invention are active against the filementous strain of *C.albicans* SC5314 with the best MIC value of 15.6 µM (12.5 µg/ml)

Said lipopeptides can be effectively used as primary agents or as adjuvants in the treatment of infectious diseases concerning different body districts, both in humans and animals. Furthermore, the lipopeptides object of the invention can also be employed in the treatment of infections of vegetal organisms.

These and other features, aspects and advantages of the present disclosure will become better understood with reference to the following description, the drawings and appended claims.

The drawings, which are incorporated in and constitute a part of this specification, illustrate embodiments of the present subject matter and, together with the description, serve to explain the principles of the disclosure. The various aspects and features described in the present disclosure can be applied, individually, wherever possible. These individual aspects, for instance the aspects and features described in the attached dependent claims, can be made subject of divisional patent applications.

It is noted that anything found to be already known during the patenting process is understood not to be claimed and to be the subject of a disclaimer.

### BRIEF DESCRIPTION OF THE DRAWINGS

- Figure 1 shows MIC obtained against a panel of Gram-positive, Gram-negative and Yeast reference strains
- Figure 2 shows MIC obtained against a clinical strain of vancomycin-resistant enterococcus faecalis, Streptococcus mutans, and Listeria monocytogenes.
- Figure 3 shows a permeabilization assay. Panel A: Gram-positive, *S.aureus*; panel B: Gram-negative, *P.aeruginosa.*

### DETAILED DESCRIPTION OF THE EMBODIMENTS

Reference will now be made in detail to the various embodiments of the invention, one or more examples of which are illustrated in the figures. Within the following description of the drawings, the same reference numbers refer to the same components. Generally, only the differences with respect to individual embodiments are described. Each example is provided by way of explanation of the invention and is not meant as a limitation of the invention. For example, features illustrated or described as part of one embodiment can be used on or in conjunction with other embodiments to yield yet a further embodiment. It is intended that the present invention includes such modifications and variations.

Before describing these embodiments, it shall be also clarified that the present description is not limited in its application to details of the construction and disposition of the components as described in the following description using the attached drawings. The present description can provide other embodiments and can be obtained or executed in various other ways. It shall also be clarified that the phraseology and terminology used here is for the purposes of description only, and cannot be considered as limitative.

All the measurements are made, unless otherwise indicated, at 25 °C and atmospheric pressure. All the temperatures, unless otherwise indicated, are expressed in degrees Centigrade.

All the ranges reported here shall be understood to include the extremes, including those that report an interval "between" two values. Furthermore, all the ranges reported here shall be understood to include and describe the punctual values included therein, and also all the sub-intervals. Moreover, all the ranges are intended as such that the sum of the values comprised therein, in the final composition, gives 100%, in particular considering that the person of skill will know how to choose the values of the ranges so that the sum does not exceed 100%.

The term "peptide", within the present invention, is defined as a multiplicity of amino acid residues linked by peptide bonds. It has the same meaning of polypeptide and can be used interchangeably. Amino acids forming a polypeptide are herein regardlessly identified by their complete name, or by their respective international official abbreviations (1- or 3-letter code).

The terms "lipopeptide" and "lipophilic conjugate" as used herein refer to a peptide covalently coupled to a fatty acid. The terms lipopeptide and lipophilic conjugate are used interchangeably throughout the specification and claims.

The conjugation can be coupled to the N-terminal of the peptide, to the C-terminal of the peptide or to any of other suitable free functional group along peptidic chain, for example, to the ε-amino group of positive charged amino acid, such as lysine.

The terms "coupling" and "conjugation" are used herein interchangeably and refer to the chemical reaction, which results in covalent attachment of a fatty acid to a peptide to yield a lipophilic conjugate.

The term "series", in the present document, is defined as all the variants of the lipopeptides of the invention, wherein the fatty acid is substituted by one selected from the group of saturated, unsaturated, monounsaturated, and polyunsaturated fatty acid and/or one or more amino acids of the peptide sequence are substituted by a homologous amino acid so that the properties of the lipopeptides are retained, even if not necessarily at the same level. Another variant can show higher or lower activity and/or a wider spectrum (for example, activity against a wider range of microbes), or be more specific for a microorganism. Preferably, conservative substitutions of amino acids are carried out in one or more amino acid residues.

According to embodiments, the present disclosure relates to a lipopeptide having a sequence FA-A-B-A'-NH2, where:
- FA represents a fatty acid selected from caproic acid (hexanoic), caprylic acid (octanoic), capric acid (decanoic), lauric acid (dodecanoic), mystiric acid (tetradecanoic), pentadecanoic acid and palmitic acid (hexadecanoic);
- unit A and A' independently consist of 1, 2 or 3 amino acids selected from the group of basic amino acids or hydrophobic amino acids ;
- unit B consist of 1, 2 or 3 amino acids, selected from the group (a) of hydrophobic amino acids and (b) the group of hydrogen-bond forming amino acids; wherein:
   (i) said hydrophobic amino acids are selected from: Ala, Phe, Ile, Leu, Pro, Tyr, Trp, Val, Met, Cys;
   (ii) said basic amino acids are selected from: Lys, His, Arg;
   (iii) said hydrogen bond-forming amino acids are selected from Asn, Gin, Ser, Thr;
-NH2 represents C-terminal amidation.In the above cited sequence, units FA, A, B, and A' are linked in the order FA-A-B-A'-NH2 to form a linear sequence.

In said structure, the units named with the same letter are not necessarily the same, but can contain different amino acids; consequently, considering the central group B, the invention includes both symmetric peptides and asymmetric ones.

Units FA represent the fatty acid selected from saturated, unsaturated, monounsaturated, and polyunsaturated fatty acids. Examples of fatty acids that can be coupled to the peptides of the present disclosure include, but not limited to, Propionic acid, Butyric acid, Valeric acid, Caproic acid, Enanthic acid, Caprylic acid, Pelargonic acid, Capric acid, Undecylic acid, Lauric acid, Tridecylic acid, Myristic acid, Pentadecylic acid, Palmitic acid, Margaric acid, Stearic acid, Nonadecylic acid, Arachidic acid, Heneicosylic acid, Behenic acid, Tricosylic acid, Lignoceric acid, Pentacosylic acid, Cerotic acid, Heptacosylic acid, Montanic acid, Nonacosylic acid, Melissic acid, Henatriacontylic acid, Lacceroic acid, Psyllic acid, Geddic acid, Ceroplastic acid , Hexatriacontylic acid, Heptatriacontanoic acid, Octatriacontanoic acid, Lignoceric acid, Palmitoleic acid, Oleic acid, Linoleic acid, Linolenic acid, Arachidonic acid, trans-Hexadecanoic acid, Elaidic acid, Lactobacillic acid, Tuberculostearic acid, and Cerebronic acid.

Units A represent the terminal regions of the peptide: unit A refers to the -NH2 terminal region, while unit A' refers to the -COOH.

In some embodiments, each unit A and A' may consist independently of 1, 2 or 3 amino acids; the type of these amino acids is not determining; however, they can be preferably selected from histidine, lysine, arginine, ornithine, alanine, cysteine, phenylalanine, methionine, isoleucine, leucine, proline, tyrosine, tryptophan and valine. Preferably, the total number of amino acids present in these two units A and A' is equal to 4, that is 2+2, 3+1 or 1+3

Units B, consist of 1,2,3 amino acids selected: a) both in the group of hydrophobic amino acids, b) and in the group of basic or hydrogen bonds-forming amino acids. Units NH2, represent the COOH-terminal amidation.

Hydrophobic amino acids of group (a) are selected from: alanine, cysteine, phenylalanine, methionine, isoleucine, leucine, proline, tyrosine, tryptophan and valine. With reference to the overall number of all the amino acids of the peptide, they preferably represent, at least, 33%.

Basic amino acids of group (b) are selected from: lysine, histidine, arginine, ornithine. Amino acids forming hydrogen bonds of group (b) are selected from asparagine, glutamine, serine, threonine.

A preferred subgroup of lipopeptides belonging to the family described above is that containing a total of 4 amino acids, wherein units A and A' consist independently of 1 amino acids, units B consist of 2 amino acids a belong to said group (a) of hydrophobic amino acids or to said group (b) of amino acids forming hydrogen bonds.

Another preferred subgroup of lipopeptides belonging to the family described above is that containing a total of 6 amino acids, wherein units A and A' consist independently of 2 amino acids, units B consist of 2 amino acids a belong to said group (i) of hydrophobic amino acids or to said group (b) of amino acids forming hydrogen bonds. Another preferred subgroup of lipopeptides belonging to the family described above is that containing a total of 9 amino acids, wherein units A and A' consist independently of 3 amino acids, units B consist of 3 amino acids a belong to said group (i) of hydrophobic amino acids or to said group (b) of amino acids forming hydrogen bonds. A more preferred subgroup is characterized in that it contains, in addition to the previously listed characteristics, 1 or 2 aromatic amino acid in unit B. Such characteristic has proven particularly useful in increasing membrane penetration of the lipopeptide, thus enhancing the antimicrobial activity.

All the amino acids present in the present peptide can be regardlessly present in D- or L-form; preferably, they are mainly (that is over 66%) or totally in the L-form. Lipopeptides according to the present invention are selected amongst the following sequences:
Cn-Arg-Ala-Trp-Arg-NH2, wherein Arg-Ala-Trp-Arg is SEQ ID No.: 1
Cn-Arg-Cys-Trp-Arg-NH2, wherein Arg-Cys-Trp is SEQ ID No.: 2
Cn-Arg-Phe-Trp-Arg-NH2, wherein Arg-Phe-Trp-Arg is SEQ ID No.: 3
Cn-Arg-Gly-Trp-Arg-NH2, wherein Arg-Gly-Trp-Arg is SEQ ID No.: 4
Cn-Arg-His-Trp-Arg-NH2, wherein Arg-His-Trp-Arg is SEQ ID No.: 5
Cn-Arg-Ile-Trp-Arg-NH2, wherein Arg-Ile-Trp-Arg is SEQ ID No.: 6
Cn-Arg-Leu-Trp-Arg-NH2, wherein Arg-Leu-Trp-Arg is SEQ ID No.: 7
Cn-Arg-Met-Trp-Arg-NH2, wherein Arg-Met-Trp-Arg is SEQ ID No.: 8
Cn-Arg-Arg-Trp-Arg-NH2, wherein Arg-Arg-Trp-Arg is SEQ ID No.: 9
Cn-Arg-Ser-Trp-Arg-NH2, wherein Arg-Ser-Trp-Arg is SEQ ID No.: 10
Cn-Arg-Val-Trp-Arg-NH2, wherein Arg-Val-Trp-Arg is SEQ ID No.: 11

Other lipopeptides described herein, which are not part of the present invention, are:
Cn-Arg-Trp-Trp-Arg-NH2, wherein Arg-Trp-Trp-Arg is SEQ ID No.: 12
Cn-Phe-Trp-Arg-Arg-NH2, wherein Phe-Trp-Arg-Arg is SEQ ID No.: 13
Cn-Arg-Arg-Phe-Trp-NH2, wherein Arg-Arg-Phe-Trp is SEQ ID No.: 14
Cn-Phe-Arg-Arg-Trp-NH2, wherein Phe-Arg-Arg-Trp is SEQ ID No.: 15
Cn-Arg-Arg-Phe-Trp-Arg-Arg-NH2, wherein Arg-Arg-Phe-Trp-Arg-Arg is SEQ ID No.: 16
Cn-Arg-Arg-Ile-Trp-NH2, wherein Arg-Arg-Ile-Trp is SEQ ID No.: 17
Cn-Ile-Trp-Arg-Arg-NH2, wherein Ile-Trp-Arg-Arg is SEQ ID No.: 18
Cn-Trp-Ile-Arg-Arg-NH2, wherein Trp-Ile-Arg-Arg is SEQ ID No.: 19
Cn-Arg-Arg-Ile-Trp-Arg-Arg-NH2, wherein Arg-Arg-Ile-Trp-Arg-Arg is SEQ ID No.: 20.

In the above sequences, n is an integer selected from 12, 14, 15, 16 such that Cn represents a fatty acid selected from: lauric acid (dodecanoic), mystiric acid (tetradecanoic), pentadecanoic acid and palmitic acid (hexadecanoic).

Another preferred aspect of the invention relates to peptides constituted of at least 80%, for example 90%, or more preferably 91-100%, for example 100%, of L-amino acids. Another preferred aspect of the invention relates to lipopeptides constituted of at least 80%, for example 90%, or more preferably 91-100%, for example 100%, of D-amino acids.

All the amino acids can be used in their natural state or in the form of their synthetic derivatives.

A preferred group of lipopeptides is that where: unit A (NH2- terminal) contains 1 amino acids, and unit A' (COOH- terminal) contains 1 amino acid, units B is represented by two hydrophobic residues.

A preferred group of lipopeptides is that where unit FA, represents a fatty acid selected from caproic acid (hexanoic), caprylic acid (octanoic), capric acid (decanoic), lauric acid (dodecanoic), mystiric acid (tetradecanoic) and palmitic acid (hexadecanoic)
According to another preferred group of peptides of the present invention, is a short peptide (up to 6-mer peptide) is coupled to a long aliphatic chain fatty acid. Thus, for example, a peptide consisting of up to six amino acid residues and having little or no antimicrobial activity is coupled to a fatty acid that consists of at least 12 carbon atoms such as, for example, lauric acid

Preferably, conjugation of a fatty acid to a peptide of the invention enhances at least one activity selected from antibacterial, antifungal, antiviral, and anticancer activity by at least 2-fold, more preferably by at least 10-fold, and most preferably by at least 20-fold. Typically, inactive antimicrobial peptide is defined as a peptide of which a concentration higher or equal than 100 µM is required to significantly inhibit bacteria or fungi growth. A peptide is defined weakly active when a concentration between 25 and 99 µM is required to significantly inhibit bacteria or fungi growth. An active peptide is defined as a peptide of which concentration between 10 and 24 µM is required to significantly inhibit bacteria or fungi growth. A peptide is defined highly active when a concentration of said peptide between 5 to 10 µM is required to significantly inhibit bacteria or fungi growth. Finally, a very high active antimicrobial peptide is defined as a peptide of which a concentration lower than 5 µM is required to significantly inhibit bacteria or fungi growth.

The present invention includes a process for the synthesis of said lipopeptides having the structure FA-A-B-A'-NH2 defined above.

Collectively, or individually, the lipopeptides of the invention are generally synthetic peptides synthesized in vitro using chemical methods known in the art. For example, they are prepared using synthetic solid phase, liquid phase, peptide condensation procedures, or any combination of the above-mentioned techniques. Amino acids composing the peptides of the invention can be natural or synthetic. Amino acids used for peptide synthesis can be amino acids wherein the alpha-amino-terminal is protected by the acid-labile group N-α-t-butyloxycarbonyl (Boc) according to the work of Merrifield (J. Am. Chem. Soc., 85: 2149-2154, 1963) or by the base-labile 9-fluorenylmethoxycarbonyl (Fmoc) as described by Carpino and Han (J. Org. Chem., 37: 3403-3409, 1972). Both Boc- or Fmoc-protected amino acids can be obtained from different commercial sources, such as, for example, Fluka, Sigma-Aldrich Bachem, Advanced Chemtech, Cambridge Biochemical Research.

In general, solid phase methods of chemical synthesis, according to M. Bodansky, Principles of peptide synthesis, (Springer-Verlag, Berlin, 1984) or J M Stewart and J D Young, Solid Phase Peptide Synthesis (Pierce Chemical Co., Rockford, Ill. 1984), consist in sequential addition of one or more amino acids to the growing peptide chain. Generally, the amine or carboxyl group of the first amino acid is protected by an optimal protecting group. The first protected amino acid is attached to a solid inert support, for example a resin. The protecting group is then removed from the residue bound to the resin and the next (properly protected) amino acids are sequentially added. After reaching the number of amino acids, all the remaining protecting groups (and any solid support) are sequentially or simultaneously removed, to give the final peptide. The fatty acid was conjugated to the N-terminus of the peptides using the same protocol used to attach protected amino acids for peptide synthesis.

It is possible to add more than one amino acid at a time to the growing chain, for example, by coupling (in suitable experimental conditions avoiding the formation of racemates due to the presence of chiral centres) a protected tripeptide with a properly protected peptide to form, after deprotection, a tetrapeptide as described, for example, by Merrifield in G. Barany and R B Merrifield, Peptides: analysis, synthesis, biology, E. and J. Gross Meienhofer eds., vol. 2 (Academic Press, New York, 1980, pp. 3-254). Said lipopeptides can be synthesized by firms providing the service of synthesis of custom peptides, for example, but not limited to, Sigma-Aldrich (St. Louis, MO, USA), SelleckChem (Houston, TX, USA), Invitrogen (Grand Island, NY, USA), Abgent (Oxfordshire, OX144RY, United Kingdom), Genscript (Piscataway, NJ, USA).

The degree of purity of the lipopeptide compound can be determined by various methods, among which by identification of HPLC peaks. Preferably, a lipopeptide producing a single peak of height and width equal to at least 75% of the input material on an HPLC column is preferred. Even more preferred is a lipopeptide producing a single peak that is at least 87%, at least 90%, at least 99% or even 99.5% of the input material on an HPLC column.

To guarantee that the lipopeptide obtained using one of the above mentioned synthetic techniques is the desired peptide for the uses or formulations described hereinafter in the present invention, analysis of the composition of the peptide is carried out with the aid of different analytical methods known in the art. The analysis of the composition can be carried out, for example, using high resolution mass spectrometry to determine the molecular weight of the peptide. Alternatively, the content of amino acids of a peptide can be confirmed hydrolyzing the lipopeptide in acid solution to identify and quantify the components of the mixture using HPLC, or an amino acid analyzer. Equally useful are thin layer chromatography methods, they can also be used to identify one or more constituent groups or residues of a desired lipopeptide.

Collectively or individually, the lipopeptides of the invention are provided with biological activity towards biota (cellular organisms belonging to the domains Archea, Bacteria or Eukaryota).

A preferential aspect of the lipopeptides of the invention relates to the lack of biological activity towards biota belonging to the domain Eukaryota, preferably biota belonging to the subkingdom Eumetazoa, even more preferably biota belonging to the phylum Chordata.

Another preferential, but not limiting, aspect of the invention relates to the percentage of water solubility of the lipopeptides of the invention, comprised between 0 and 2 mg/ml, preferably between 2.1 and 10 mg/ml, even more preferably between 10.1 and 14 mg/ml, for example equal or greater than 15 mg/ml.

Collectively or individually, the lipopeptides of the invention are provided with antimicrobial activity for a wide range of pathogens such as, for example, bacteria, fungi, yeasts, parasites, protozoa and viruses. A "pathogenic agent" is generally defined as any (uni- or multi-cellular, or having subcellular organization) organism causing a disease in an organism.

Collectively or individually, the antimicrobial lipopeptides of the invention are readily synthesized, highly efficient, proteolytically stable, essentially salt-insensitive, with low, if any, hemolytic and/or cytotoxic activity towards Eukaryota
The mass of the lipopeptides of the invention can be determined through procedures known in the art, for example, mass spectroscopy. Essentially, this technique includes ionization of a compound and subsequent separation of the ions produced based on their own mass/charge ratio. Mass spectrometers have different methods to determine the mass/charge ratio of the ion, for example the time of flight. Essentially, ions coming from the source are accelerated by a potential (V) to the same kinetic energy, then are allowed to fly along a tube towards a detector. If one spectrometer has a flight tube of a length L, the time of flight for a given ion is given by: t=(L2^{∗}m/2^{∗}z^{∗}e^{∗}V). Starting from the m/z ratio, function of the residence time in the flight tube, the mass of a given ion can be calculated. The flight tube, though, has low resolution (low ability to discriminate two ions with similar m/z), to overcome this, most of these analyzers are provided with a "Reflectron" i.e. one mirror able to reflect ions and then to make the ion cover twice the path. In this way, it is possible to discriminate between 2 ions having very similar times of flight in the flight tube. The use of the "Reflectron" narrows the range of molecular mass that can be analyzed, that is comprised between 200 and 5000-10000 Da.

The structure of lipopeptides in solution can be analyzed by a technique known in the art, called circular dichroism. It is part of the chiroptical spectroscopies, that is those spectroscopic techniques that, using polarized light, emphasize the optical, activity of test molecules. Results obtained by this technique provide information on the percentages of secondary structures present in polypeptides. Although it is not possible to establish the position in the sequence, this technique can be used for a first screening for the choice of the solvent system for NMR analysis and as a check of the results obtained from computational calculation.

The antimicrobial activity of a lipopeptide can be determined using a method known in the art, for example, the broth-dilution method. Essentially, this method involves the growth of a microorganism in a liquid medium until it reaches the logarithmic phase. The test lipopeptide is serially diluted with growth medium for the test bacterium in the wells of a multiwell plate. An optimal concentration of the microorganism is added to the wells containing the serially diluted lipopeptide. The plate is incubated in a thermostat at a temperature of 37° C. for a time sufficient for the microorganism to grow. The growth of the microorganism, evaluated in comparison with a negative control (microorganism grown in the absence of the lipopeptide), is determined by detecting the absorbance of the solution containing the bacterium, for example, at 605 nm.

A further method to determine if a lipopeptide has antimicrobial activity consists in verifying the existence of a damage to the bacterial membrane. This method essentially consists in contacting, in a liquid medium, the microorganism with a lipopeptide. To the liquid medium, a molecule able to be excluded from the intact membrane of the microorganism is added. Such molecule is the propidium iodide (PI) solution, a bacterial fluorescence staining dye that can be applied for microbial cell viability assay in different principles. Briefly, PI is an ethidium bromide analog that emits red fluorescence upon intercalation with double-stranded DNA. Though PI does not permeate viable cell membranes, it passes through injured cell membranes and stains the nuclei. PI is often used in combination with a fluorescein compound, for simultaneous staining of viability and membrane injury.

In the present invention, cytotoxicity of the lipopeptides was also determined, for example, by determining hemolysis of red blood cells and scoring the antimicrobial lipopeptides based on their minimum hemolytic concentration. In the present document, MHC10 is defined as the concentration of lipopeptide that determines 10% of hemolysis, MHC50 is the concentration of lipopeptide that determines 50% of hemolysis and MHC90 is the concentration of lipopeptide that determines 90% of hemolysis. Lipopeptides that at the concentration of 100 µg/ml fell in the class defined as MHC10 were selected.

In the present invention, cytotoxicity of the lipopeptides against a epithelial cell line was also determined, for example, A549 cells (lung cancer). Cells were cultured in the presence and in the absence of peptides and the damage to the membrane was evaluated by the vital stain MTT.

A preferred aspect of the invention relates to the use of a lipopeptide having the FA-A-B-A'-NH2 structure defined above, for manufacturing a medicament useful for the treatment of a subject affected by an infection caused by bacteria, fungi and/or yeasts; the invention additionally comprises the same lipopeptide for use in the treatment of a subject affected by an infection caused by bacteria, fungi and/or yeasts. In a first variant, the treatment is particularly directed against the group of Gram-positive bacteria; in a second variant, the treatment is directed against the group of Gram-negative bacteria; in a third variant, the treatment is directed against the group of fungi and yeasts; in a fourth variant, the treatment is directed against microorganisms belonging to more than one of said groups. The term "treatment" refers to the effects of the lipopeptides of the invention able to provide benefit to patients affected by an infectious disease, for example an improvement of the conditions of the patient or a delay in the progression of the disease. In the present document, term "infection", or its synonym "infectious disease" means the invasion, colonization and/or multiplication of a microorganism inside or on another host organism. The term "microbial infection" means an infectious disease caused by a pathogenic agent, as previously defined, for example, a bacterium, a parasite, a protozoan, a virus or a fungus, comprising yeasts. In the present document, the term "subject" defines any multicellular organism, among which a human being, animal, plant or insect that can be infected by a microorganism. Preferably, the subject is any animal organism, for example a human being or an animal, that can be infected by a microorganism against which an antimicrobial lipopeptide or a variant thereof is active.

A pathogenic bacterium, as previously defined, can originate from one of the bacterial species selected in the group including: Staphylococcus spp., for example, *Staphylococcus aureus* (e.g. *Staphylococcus aureus* ATCC 25923), Enterococcus spp., for example, *Enterococcus faecalis* ATCC 29212; Pseudomonas spp., for example *Pseudomonas aeruginosa* ATCC 27853; Mycobacterium spp., for example *Mycobacterium tuberculosis;* Enterobacter spp.; Campylobacter spp. ; Salmonella spp. (e.g. *Salmonella enteritidis* ATCC 13076); Streptococcus spp., for example Streptococcus group A or B, *Streptoccocus pneumoniae, Streptococcus suis, Streptococcus mutans* ATCC 35668, Helicobacter spp., for example *Helicobacter pylori;.* Neisseria spp., for example *Neisseria gonorreae, Neisseria meningitidis; Borrelia burgdorferi,* Shigella spp., for example, Shigella flexneri; *Escherichia coli* ATCC 25922; Haemophilus spp., for example *Haemophilus influenzae; Francisella tularensis,* Bacillus spp., for example *Bacillus anthracis;* Clostridium spp., *Clostridium botulinum, Clostridium perfringens,* Yersinia spp., for example, *Yersinia pestis;* Treponema spp.; Burkholderia spp.; for example *Burkholderia cepacia* ATCC 17759, *B. mallei* and *B. pseudomallei*; Stenotrophomonas spp., for example *Stenotrophomonas maltophilia* ATCC 13637, Listeria spp., for example *Listeria monocytogenes* (DSM 20600).

The biological activity of the lipopeptides of the invention against microorganisms was determined, for example, through broth-microdilution assay and count of the colonies in a plate. In the present invention, the ability of the lipopeptides to reduce or prevent the growth of those bacteria involved in primarily clinically relevant infectious diseases was determined. For example, the activity on Gram-positive bacteria was verified with reference to bacteria such as *S. aureus.*

*Staphylococcus aureus* is a gram-positive, round-shaped bacterium that is a member of the Firmicutes, and is frequently found in the nose, respiratory tract, and on the skin. It is often positive for catalase and nitrate reduction and is a facultative anaerobe that can grow without the need for oxygen. Although *S. aureus* is not always pathogenic, it is a common cause of skin infections including abscesses, respiratory infections such as sinusitis, and food poisoning. Pathogenic strains often promote infections by producing virulence factors such as potent protein toxins, and the expression of a cell-surface protein that binds and inactivates antibodies. The emergence of antibiotic-resistant strains of *S. aureus* such as methicillin-resistant *S. aureus* (MRSA) is a worldwide problem in clinical medicine. Despite much research and development there is no approved vaccine for *S. aureus.*

For example, the activity on Gram-negative bacteria was verified with reference to bacteria such as *Pseudomonas aeruginosa and Escherichia coli*
*Pseudomonas aeruginosa* is a problematic Gram-negative bacterium by its invasivity and heterogeneous resistance to antibacterial chemotherapies. This microorganism is responsible of severe infections and is the cause of severe morbidity in immunocompromised subject due to viral infections such as HIV, anticancer chemotherapy or immunosuppressive therapies. Additionally, this bacterium is often the etiological agent of severe infectious diseases of lower respiratory tract, urinary tract, cutaneous lesions (wounds, ulcers) in in-house juvenile population, including subjects affected from cystic fibrosis, and hospitalized patients. In the last years, the incidence of infections by Pseudomonas, in cystic fibrosis, has been dramatically increasing. *Escherichia coli* is a Gram-negative microorganism belonging to the family of Enterobacteriaceae, to which bacteria such as Shigella, Salmonella, Klebsiella or Proteus belong. Escherichia coli is an important pathogenic agent often causing infectious diseases of the urinary tract, bacteremias, hospital and community acquired pneumonia and various infectious diseases of the abdominal cavity. The emerging resistance to antibacterial chemotherapies observed in the last years in Escherichia coli is becoming a serious health problem. Of particular interest is the resistance related to the production of wide spectrum beta-lactamases that has made this bacterium resistant to cephalosporins and fluoroquinolones, in particular to ciprofloxacin.

A fungal pathogen can originate from one of the fungi (comprising yeasts) belonging to the group including genera Candida spp. (for example C. albicans), Epidermophyton spp. Exophiala spp., Microsporum spp., Trichophyton spp. (for example T. rubrum and T. interdigitale), Tinea spp., Aspergillus spp., Blastomyces spp., Blastoschizomyces spp., Coccidioides spp., Cryptococcus spp. (for example Cryptococcus neoformans), Histoplasma spp., Paracoccidiomyces spp., Sporotrix spp., Absidia spp., Cladophialophora spp., Fonsecaea spp., Phialophora spp., Lacazia spp., Arthrographis spp., Acremonium spp., Actinomadura spp., Apophysomyces spp., Emmonsia spp., Basidiobolus spp., Beauveria spp., Chrysosporium spp., Conidiobolus spp., Cunninghamella spp., Fusarium spp., Geotrichum spp., Graphium spp., Leptosphaeria spp., Malassezia spp. (for example Malassezia furfur), Mucor spp., Neotestudina spp., Nocardia spp., Nocardiopsis spp., Paecilomyces spp., Phoma spp., Piedraia spp., Pneumocystis spp., Pseudallescheria spp., Pyrenochaeta spp., Rhizomucor spp., Rhizopus spp., Rhodotorula spp., Saccharomyces spp., Scedosporium spp., Scopulariopsis spp., Sporobolomyces spp., Syncephalastrum spp., Trichoderma spp., Trichosporon spp., Ulocladium spp., Ustilago spp., Verticillium spp., Wangiella spp. The biological activity of lipopeptides of the invention against a fungus or a yeast was determined for example, by broth-microdilution assay and count of the colonies in a plate. For example, the ability of an isolated lipopeptide or of one belonging to a library of lipopeptides to inhibit the growth and/or kill fungi (comprising yeasts) belonging to the genus Candida spp. or Malassezia spp., for example, Candida albicans, was determined.

*Candida albicans* is a type of yeast that is commonly used as a model organism for biology. It is generally referred to as a dimorphic fungus since it grows both as yeast and filamentous cells. It is a common member of human gut flora and does not seem to proliferate outside mammalian hosts. It is detectable in the gastrointestinal tract and mouth in 40-60% of healthy adults. It is usually a commensal organism, but can become pathogenic in immunocompromised individuals under a variety of conditions. It is one of the few species of the *Candida* genus that cause the infection candidiasis in humans. Overgrowth of the fungus results in candidiasis (candidosis). Candidiasis is for example often observed in HIV-infected patients. *C. albicans* is the most common fungal species isolated from biofilms either formed on (permanent) implanted medical devices or on human tissue. *C. albicans,* together with *C. tropicalis, C. parapsilosis* and *C. glabrata,* is responsible for 50-90% of all cases of candidiasis in humans. A mortality rate of 40% has been reported for patients with systemic candidiasis due to *C. albicans.* Estimates range from 2800 to 11200 deaths caused annually in the USA due to *C. albicans* causes candidiasis.

Collectively, or individually, the lipopeptides of the present invention can be useful also in the treatment of infections generally associated with the skin among which, and not limited to, ulcers and lesions, skin wounds, cuts or burns.

A further preferred aspect of the invention reports that the lipopeptides, collectively or individually, are useful in the treatment of bacterial cutaneous infections or pyodermites.

Another aspect includes the collective or individual use of the lipopeptides of the invention in the treatment of (clinical or surgical) diseases complicated by bacterial superinfections among which, and not limited to, infections associated to mucosae, infections associated to gastrointestinal, urogenital tract, infections of the urinary tract (for example pyelonephritis, cystitis, urethritis) or respiratory infections, for example, cystic fibrosis.

Mammals and, in general, other animals can be treated with the lipopeptides described in the present invention. Mammals and birds comprise, but they are not limited to, humans, dogs, cats and bird pets and productive livestock, such as horses, cattle, sheep, goats, pigs, chicken and turkeys and poultry.

A second preferred aspect includes the use of the lipopeptides of the invention in the treatment of infectious diseases: infections by S.aureus, Klebsiella, Salmonella, Yersinia, Proteus, Colibacilli, Clostridium spp. and Streptococcus spp. of pet animals and production livestock.

Another preferred aspect relates to the treatment of glanders in equids and melioidosis in carnivores and infections by Pseudomonas aeruginosa in pet animals and production livestock.

A further aspect relates to the treatment of infections by Bordetella spp., in pet animals and production livestock; infections by Moraxella spp.; infections by Francisella spp., infections by Brucella spp., infections by Campylobacter spp., infections by Pasteurella spp.; infections by Actinobacillus spp. (actinobacillosis); infections by Haemophilus spp.; infections by Streptococcus spp. (among which mastitis in cows, strangles); infections by Staphylococcus spp. (among which mastitis, pyodermitis, endometritis); infections by Bacillus spp., among which anthrax; infections by Clostridium spp., among which tetanus, botulism and symptomatic anthrax; infections by Listeria spp. (listeriosis); infections by Erysipelothrix spp., among which erysipeloid; infections by Leptospira spp., Serpulina (superficial necrotic enteritis), Treponema spp. (sifilis of the rabbit), Borrelia spp. in pet animals and production livestock.

Eventually, also plants can be treated with the lipopeptides of the invention.

The present invention provides pharmaceutical compositions comprising the lipophilic conjugates of the invention and a cosmetically and/or pharmaceutically acceptable carrier. The term "pharmaceutically acceptable carrier" refers to a vehicle which delivers the active components to the intended target and which does not cause harm to humans or other recipient organisms. As used herein, "pharmaceutical" will be understood to encompass both human and animal pharmaceuticals. Useful carriers include, for example, water, acetone, ethanol, ethylene glycol, propylene glycol, butane-1, 3-diol, isopropyl myristate, isopropyl palmitate, or mineral oil.

Methodology and components for formulation of pharmaceutical compositions are well known in art, and can be found, for example, in A.J. Winfield, R.M.E. Richards, Pharmaceutical Practice second edition, Churchill Livingstone London (1998*)* or in P. Colombo, P.L. Catellani, A. Gazzaniga, E. Menegatti, E. Vidale Principi di Tecnologie Farmaceutiche, Casa Editrice Ambrosiana, Milano (2015*).* The pharmaceutical composition may be formulated in any form appropriate to the mode of administration, for example, solutions, colloidal dispersions, emulsions (oil-in-water or water-in-oil), suspensions, creams, lotions, gels, foams, sprays, aerosol, ointment, tablets, suppositories, and the like.

The pharmaceutical compositions can also comprise other additional materials, which may be chosen depending on the carrier and/or the intended use of the composition. Additional components include, but are not limited to, chelating agents e.g. EDTA, EGTA, emulsion stabilizers, e.g., ethylcellulose, preservatives, e.g., methyl paraben, fragrances, humectants, e.g., glycerin or propylene glycol or sorbitol, waterproofing agents, water soluble film-formers, e.g., hydroxypropyl methylcellulose, oil-soluble film formers, cationic or anionic polymers, and the like.

The pharmaceutical compositions useful in the practice of the present invention comprise a lipopeptide of the invention optionally formulated into the pharmaceutical composition as a pharmaceutically acceptable salt form. Pharmaceutically acceptable salts include the acid addition salts (formed with the free amino groups of the polypeptide), which are formed with inorganic acids, such as for example, hydrochloric or phosphoric acid, or with organic acids such as acetic, oxalic, tartaric, and the like. Suitable bases capable of forming salts with the lipopeptides of the present invention include, but are not limited to, inorganic bases such as sodium hydroxide, ammonium hydroxide, potassium hydroxide and the like; and organic bases such as mono-, di- and tri-alkyl and aryl amines (e.g. triethylamine, diisopropyl amine, methyl amine, dimethyl amine and the like) and optionally substituted ethanolamines (e.g.ethanolamine, diethanolamine and the like).

Collectively, or individually, the lipopeptides of the invention can also be formulated as solutions for oral administration or as solutions suitable for parenteral administration, such as, for example by intramuscular, subcutaneous, intraperitoneal or intravenous route.

The pharmaceutical formulations of the lipopeptides of the invention can also be in the form of an aqueous solution, a dry form or a dispersion, or alternatively in the form of an emulsion, a suspension, a cream, a salve or an ointment.

Collectively, or individually, the lipopeptides of the present invention can be included in suspensions, solutions or oily emulsions in vehicles or in water and can contain useful suspending, stabilizing agents and/or agents promoting dispersion.

Collectively, or individually, the lipopeptides of the present invention can be formulated in the form of a powder, obtained by aseptic isolation of a sterile solid or by freeze-drying a solution to be reconstituted in the form of a solution with the aid of a suitable vehicle before use, for example, water for injectable preparations.

Collectively, or individually, the lipopeptides of the present invention can also be administered via the respiratory tract. For administration by inhalation or insufflation, the composition can be in the form of a dry powder, for example, a mixed powder of therapeutic agent and a suitable powdered basis, such as lactose or starch.

Collectively, or individually, the lipopeptides of the present invention can be administered in an aqueous solution in the form of aerosol or by inhalatory route.

Throughout the description and claims of this specific invention, the words "comprise" and "contain" and variations of the words, for example "comprises" and "contains", mean "comprising but not limited to", and do not mean to exclude other fractions, additives, components or whole steps.

Throughout the description and claims of this specific invention below, the singular includes the plural, unless the context requires differently. Additionally, in those parts where the indefinite article is used, it is specifically to be meant as plurality and singularity, unless the context requires differently.

With "individually" or "individual" is meant that the invention comprises the mentioned antimicrobial lipopeptide or groups of antimicrobial peptides, and that, although the single lipopeptides or groups of lipopeptides may not be listed separately here, the claims listed below can define such peptides or groups of lipopeptides in a divisible way and separately the ones from the others.

With "collectively" or "collective" is meant that the invention includes any number or combination of mentioned antimicrobial lipopeptides or groups of antimicrobial peptides, and that, it being understood that such numbers or combinations of peptides or groups of peptides cannot be specifically cited in the present document, the claims listed below can define such combinations or sub-combinations separately and in a divisible way from any other combination of lipopeptides or groups of lipopeptides.

The present invention is further described in the following and not limiting examples.

### EXPERIMENTAL PART AND EXAMPLES

### Synthesis of Lipopeptides

### Method

Synthesis of the lipopeptides was performed by the company GenScript, through its associate in the United States. The synthesis was carried out by techniques known in the art and specifically by solid phase synthesis. The purified lipopeptides, which were shown to be homogeneous (∼95%) by analytical HPLC, were subjected to amino acid analysis and electrospray mass spectroscopy to confirm their composition and molecular weight. The fatty acid was conjugated to the N-terminus of the peptides using the same protocol used to attach protected amino acids for peptide synthesis.

### Results

Twenty lipopeptides were synthesized, having length of 4-6 amino acids and a degree of purity comprised between 85 and 98%. The lipopeptides were delivered in cryovials containing the remaining at least 1.5 mg of lipopeptides. In table below it was shown the sequence, molecular weight and retention time of lipopeptides.

| **ID** | **Sequence** | **MW (theoretical)** | **Retention Time (min)** |
|---|---|---|---|
| 1 | Lau-RAWR-NH2 | 768.60 (768.99) | 18.931 |
| 2 | Lau-RCWR-NH2 | 801.00 (801.07) | 22.492 |
| 3 | Lau-RFWR-NH2 | 845.30 (845.11) | 23.592 |
| 4 | Lau-RGWR-NH2 | 755.10 (754.99) | 18.415 |
| 5 | Lau-RHWR-NH2 | 835.30 (835.07) | 21.476 |
| 6 | Lau-RIWR-NH2 | 810.85 (811.09) | 20.108 |
| 7 | Lau-RLWR-NH2 | 811.20 (811.09) | 23.079 |
| 8 | Lau-RMWR-NH2 | 829.50 (829.13) | 12.393 |
| 9 | Lau-RRWR-NH2 | 854.12 | 21.651 |
| 10 | Lau-RSWR-NH2 | 785.40 (785.01) | 16.117 |
| 11 | Lau-RVWR-NH2 | 797.10 (797.60) | 19.317 |
| 12 | Lau-RWWR-NH2 | 884.14 | 9.537 |
| 13 | Lau-FWRR-NH2 | 845.40 (845.09) | 25.148 |
| 14 | Lau-RRFW-NH2 | 845.10 (845.11) | 20.957 |
| 15 | Lau-FRRW-NH2 | 845.30 (845.11) | 20.619 |
| 16 | Lau-RRFWRR-NH2 | 1157.60 (1158.56) | 22.087 |
| 17 | Lau-RRIW-NH2 | 811.10 | 22.629 |
| 18 | Lau-IWRR-NH2 | 811.10 | 14.053 |
| 19 | Lau-WIRR-NH2 | 811.10 | 24.361 |
| 20 | Lau-RRIWRR-NH2 | 1123.46 | 18.060 |

### Example 1

### Effect of Lipid Length on the antimicrobial activity.

### Methods

The minimum inhibitory concentration values (MICs) of the lipopeptides and of conventional antibiotics were determined by the broth microdilution susceptibility test following the guidelines of the NCCLS with mid-log phase cultures. Serial two-fold dilutions of each peptide were prepared (final volume of 50 ul) in 96-well polypropylene microtiter plates (UNIFO, Treviso, Italy) with MH broth for bacteria and RPMI-1640 for fungi. Each dilution series included con-trol wells without peptide. A total of 50 ul of the adjusted inoculum (approximately 5 × 10 5 cells/ml for bacteria or 5 × 10 4 cells/ml for fungi, in the appropriate medium) was added to each well. To evaluate the MIC, microtiter plates with bacteria were incubated at 37° C overnight, while those with fungi were incubated at 30° C for 48 h. The fatty acid tested were: C6 (hexanoic),C8 (octanoic),C10 (decanoic),C12 (lauric),C14 (mystiric).

### Results

The result in Table below show that the antimicrobial activity is related to chain length up to lauric acid (C12, also indicated as "Lau" in the present description and attached rawings). The conjugation with Lauric acid strongly enhances the antimicrobial activity against all bacteria tested ranging from 4 up to 128 fold for *S.aureus;* 4 up to 32 for *E.coli* and *P.aeruginosa.*

| | | *Parameters* | | | *Bacteria (MIC, ug*/*ml)* | | |
|---|---|---|---|---|---|---|---|
| **ID** | **Sequenc e** | **MW** | **Retention time (min)** | **Fatty acid** | **S.aureus** | **P.aeruginos a** | **E.coli** |
| 0 | RFWR | 645.7 | ND | --- | >400 | >400 | >400 |
| 1 | RFWR | 762.0 | 18.675 | C6 | >400 | >400 | >400 |
| 2 | RFWR | 788.6 | 17.974 | C8 | 100 | 100 | 100 |
| 3 | RFWR | 817.0 | 22.639 | C10 | 12.5 | 50 | 25 |
| 4 | **RFWR** | **845.3** | **23.592** | **C12** | **3.125** | **12.5** | **12.5** |
| 5 | RFWR | 873.0 | 26.654 | C14 | 6.125 | 50 | 25 |

### Example 2

### Membrane Permeabilization Assay

### Methods

Flow cytometric assays were based on detection of increased permeability of bacterial and fungal cells to propidium iodide (PI), a membrane impermeant DNA-intercalating dye, following treatment with lipopeptides. Analyses were performed with a Cytomics FC 500 (Beckman-Coulter, Inc., Fullerton, CA, USA) equipped with an argon-cooled argon laser (488 nm, 5 mW) and standard system configuration for orange-filtered light detection (620 nm). For these analyses, *S.aureus* or *P.aeruginosa* were diluted in Mueller-Hinton medium to give 1 · 10⁶ cells/mL, and aliquots of the bacterial suspension were then incubated with the lipopeptides at 37°C for different times (15,30 and 60 minutes). A 0.2 mm-filtered solution of PI (Sigma-Aldrich) was then added to the peptide-treated fungi at a final concentration of 1 mg/L, and samples were acquired after 4 min of incubation at 30° C. To obtain a positive control for permeabilization, bacterial suspensions were pelleted and resuspended in cold absolute ethanol for 30 min at -20°C. Ethanol was removed by aspiration following centrifugation at 1000 g for 10 min, and the pellet resuspended in Mueller-Hinton medium. All experiments were conducted in triplicate. Data analysis was performed with the WinMDI software (Dr J. Trotter, Scripps Research Institute, La Jolla, CA, USA). Data are expressed as means ± SD.

### Results

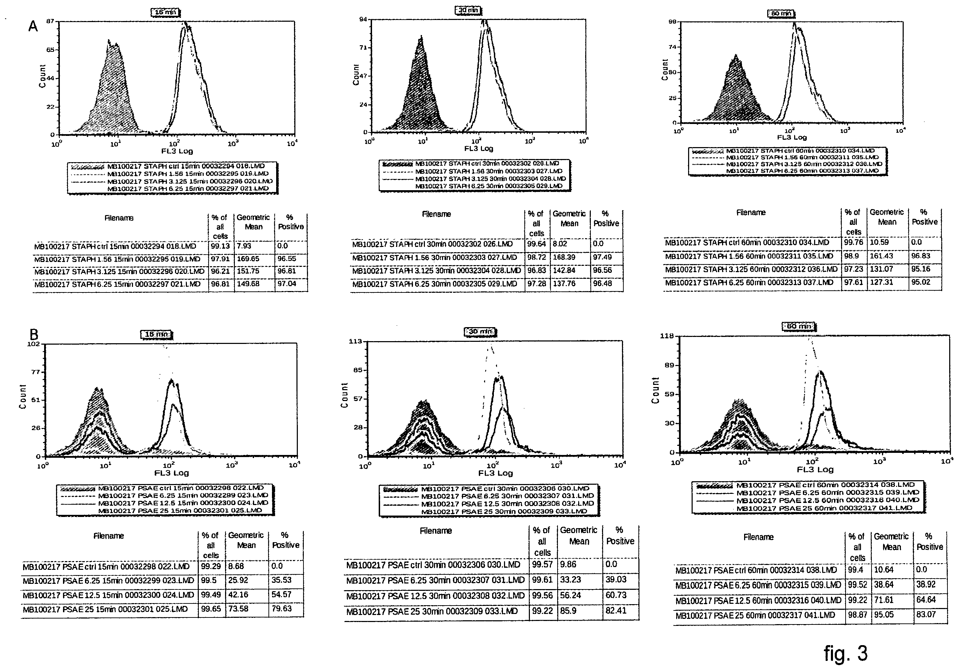

The result (Figure 3, panel A: Gram-positive, *S.aureus*; panel B: Gram-negative, *P.aeruginosa*) clearly show that peptide are highly efficient in permeabilizing the bacterial membrane (e.g. *S.aureus,* 15 min more than 95%). The permeabilization is less pronounced in the case of Gram-negative bateria (e.g. P.aeruginosa, 15 min ∼80% of bacterial cells are permeabilized)

### Example 3

### Determination of the spectrum of activity of antimicrobial lipopeptides on Gram-Positive Bacteria

### Methods

The minimum inhibitory concentration values (MICs) of the lipopeptides and of conventional antibiotics were determined by the broth microdilution susceptibility test following the guidelines of the NCCLS with mid-log phase cultures. Serial two-fold dilutions of each peptide were prepared (final volume of 50 ul) in 96-well polypropylene microtiter plates (UNIFO, Treviso, Italy) with MH broth for bacteria and RPMI-1640 for fungi. Each dilution series included con-trol wells without peptide. A total of 50 ul of the adjusted inoculum (approximately 5 × 10 5 cells/ml for bacteria or 5 × 10 4 cells/ml for fungi, in the appropriate medium) was added to each well. To evaluate the MIC, microtiter plates with bacteria were incubated at 37° C overnight, while those with fungi were incubated at 30° C for 48 h.

### Results

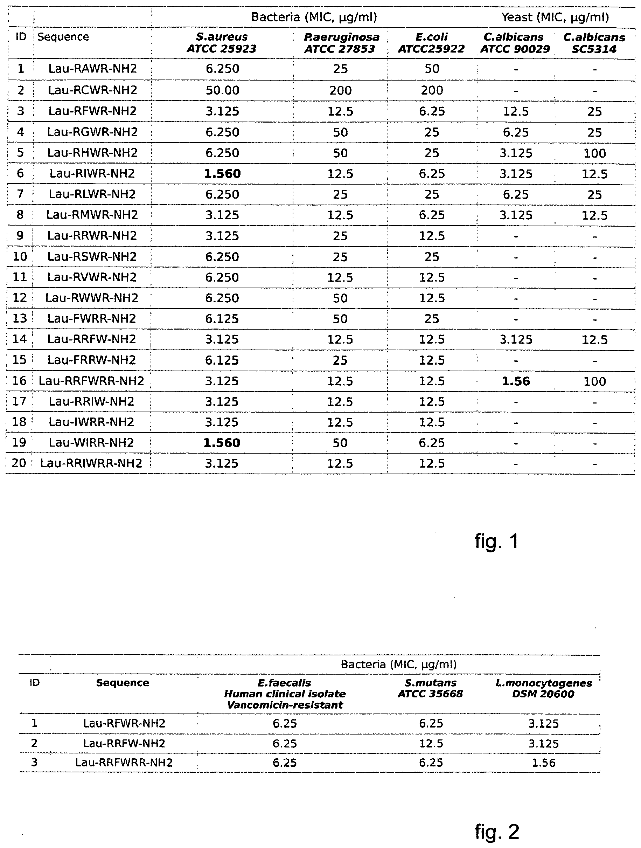

In Figure 1, MIC values for the different lipopeptides are expressed. Lipopeptide 6 results to be very highly active against *S.aureus* with an MIC value of 1.56 µg/ml (1.77 µM). The remaining lipopeptide have a MIC value ranging from 3.66 to 59 µM. Moreover in Figure 2, it was shown the activity of the best lipopeptides against a clinical strain of Vancomicin-resistant *Enterococcus fecalis* long with *S. mutans* and *Listeria monocytogenes.*

### Example 4

### Determination of the spectrum of activity of antimicrobial lipopeptides on Gram-Negative Bacteria

### Methods

The minimum inhibitory concentration values (MICs) of the lipopeptides and of conventional antibiotics were determined by the broth microdilution susceptibility test following the guidelines of the NCCLS with mid-log phase cultures. Serial two-fold dilutions of each peptide were prepared (final volume of 50 ul) in 96-well polypropylene microtiter plates (UNIFO, Treviso, Italy) with MH broth for bacteria and RPMI-1640 for fungi. Each dilution series included control wells without peptide. A total of 50 ul of the adjusted inoculum (approximately 5 × 10 5 cells/ml for bacteria or 5 × 10 4 cells/ml for fungi, in the appropriate medium) was added to each well. To evaluate the MIC, microtiter plates with bacteria were incubated at 37° C overnight, while those with fungi were incubated at 30° C for 48 h.

### Results

In Figure 1, MIC values for the different lipopeptides are expressed. Lipopeptides 3, 6 and 14 result to be highly active against *E. coli* with an MIC value of 6.25 µg/ml (7.39 µM), 6.25 µg/ml (7.39 µM), 12.5 µg/ml (14.7 µM), respectively. Relative to P. *aeruginosa,* lipopeptides 3, 6 and 14 result to be active with a MIC value of 12.5 µg/ml (14.7 µM)

### Example 5

### Antimicrobial activity of lipopeptides against fungal strains (Yeasts)

### Methods

The minimum inhibitory concentration values (MICs) of the lipopeptides and of conventional antibiotics were determined by the broth microdilution susceptibility test following the guidelines of the NCCLS with mid-log phase cultures. Serial two-fold dilutions of each peptide were prepared (final volume of 50 ul) in 96-well polypropylene microtiter plates (UNIFO, Treviso, Italy) RPMI-1640 for fungi. Each dilution series included con-trol wells without peptide. A total of 50 ul of the adjusted inoculum (approximately 5 × 10 5 cells/ml for bacteria or 5 × 10 4 cells/ml for fungi, in the appropriate medium) was added to each well. To evaluate the MIC, microtiter plates with bacteria were incubated at 37° C overnight, while those with fungi were incubated at 30° C for 48 h.

### Results

This example was performed on two different strain of *C.albicans.* Both strain are human clinical isolate recovered from a patient with generalized candidiasis. The strain SC5314 was highly filamentous *in vitro* and more invasive *in vivo,* while the ATCC strain showed less virulence, pseudo-hyphe and superficial invasion. Figure 1 showed that peptide Lau-RRFWRR-NH2 had the best MIC against ATCC strain with a value of 1.56 µg/ml, while Lau-RIWR-NH2, Lau-RMWR-NH2 and Lau-RRFW-NH2 had a MIC value of 12.5 µg/ml against the SC5314 strain

### Example 6

### Cytotoxicity of the antimicrobial lipopeptides

This example evaluates the cytotoxicity of antibacterial lipopeptides by observing in the presence and in the absence of lipopeptide.

### Methods

The colorimetric 3-(4,5-dimethylthiazol-2-yl)-2,5-diphenyl tetrazolium bromide (MTT) assay was performed to assess the metabolic activity of cells plated into 96-well culture plates (10 5 cell/well) and treated with 0.5-25 µM of in complete medium for 24-72 h. At the end of treatments, fullerenes containing medium were removed and replaced with fresh medium; for the cytotoxic assay, 20 µL stock MTT (5 mg/mL) was added to each well, and cells were then incubated for 4 hr at 37 °C. The converted MTT dye was solubilised with acidic isopropanol (0.04 N HCl in absolute isopropanol). Absorbance was measured at 540 nm and 630 nm using a microplate reader (Automated Microplate Reader EL311, BIO-TEK Instruments, Vermont, USA). All measurements were done in triplicate and each experiment was repeated at least three times.

### Results

Cytotoxicity of the compounds that displayed the best antimicrobial activity (lipopeptide 3 and 6) was evaluated. Peptide 3 and 6 showed LC50 (100 µg/ml, ∼118 µM) values higher than the MIC obtained for *S.aureus* (3.66 and 1.77 µM, respectively) and for *E.coli* (7.39 µM for both lipopeptides).

## Claims

1. A lipopeptide having a sequence FA-A-B-A'-NH2, wherein:
• FA represents a fatty acid selected from caproic acid (hexanoic), caprylic acid (octanoic), capric acid (decanoic), lauric acid (dodecanoic), mystiric acid (tetradecanoic), pentadecanoic acid and palmitic acid (hexadecanoic);
• unit A and A' independently consist of 1, 2 or 3 amino acids selected from the group of basic amino acids or hydrophobic amino acids;
• unit B consist of 1, 2 or 3 amino acids, selected from the group (a) of hydrophobic amino acids and (b) the group of hydrogen-bond forming amino acids; wherein:
(i) said hydrophobic amino acids are selected from: Ala, Phe, Ile, Leu, Pro, Tyr, Trp, Val, Met, Cys;
(ii) said basic amino acids are selected from: Lys, His, Arg;
(iii) said hydrogen bond-forming amino acids are selected from Asn, Gin, Ser, Thr;
• -NH2 represents C-terminal amidation;
wherein A-B-A' is a sequence selected from the group of the following sequences: SEQ ID No.: 1, SEQ ID No.: 2, SEQ ID No.: 3, SEQ ID No.: 4, SEQ ID No.: 5, SEQ ID No.: 6, SEQ ID No.: 7, SEQ ID No.: 8, SEQ ID No.: 9, SEQ ID No.: 10, SEQ ID No.: 11.

2. The lipopeptide according to claim 1, wherein the substructure A-B-A' contains from 2 to 4 points of alternation between amino acids of group (a) and group (b), or vice versa.

3. The lipopeptide according to claim 1 or 2, wherein at least one-third of said aminoacids are selected from the group of proteinogenic, non-proteinogenic or non-standard amino acids.

4. The lipopeptide according to claim 1, 2 or 3, wherein at least one amino acid is selected from the group of D-amino acids.

5. The lipopeptide according to anyclaims from 1 to 4, wherein FA is defined as Cn, wherein n is an integer selected from 12, 14, 15, 16 such that Cn represents a fatty acid selected from: lauric acid (dodecanoic), mystiric acid (tetradecanoic), pentadecanoic acid and palmitic acid (hexadecanoic).

6. The lipopeptide according to claim 1, wherein n is 12, such that FA is Lauryl.

7. A composition comprising at least one lipopeptide according to any claims hereinbefore and a pharmaceutically acceptable carrier.

8. The composition according to claim 7, wherein said lipopeptide is present in a concentration ranging from about 1 µg/mL to about 12.5% (w/v).

9. The composition according to claims 7 or 8, wherein said composition is in the form of an aerosol, cosmetic preparation, spray, emulsion, liquid, lotion, cream, paste, ointment, powder or foam.

10. The composition according to claim 9 for use in the prevention or treatment of skin infections caused by bacteria or fungi, ear infections caused by bacteria or fungi, bacterial or fungal vaginosis.

11. The composition of claim 9 for use in the prevention or treatment of infections of vegetal organisms.

12. A composition according to any claims from 9 to 11 for use in the prevention or treatment of a microbial infection of the skin or mucosal tissue of a mammal by administering to the infected area of said mammal a therapeutically effective amount of said composition according to any claims from 9 to 11.

13. The composition for use according to claim 12, wherein said therapeutically effective amount of the composition comprises said polipeptide at a concentration ranging from about 1 µg/mL to about 12.5% (w/v).

## Patentansprüche

1. Ein Lipopeptid aufweisend eine Sequenz FA-A-B-A'-NH2, wobei:
• FA eine Fettsäure repräsentiert, die aus Capronsäure (Hexansäure), Caprylsäure (Octansäure), Caprinsäure (Decansäure), Laurinsäure (Dodecansäure), Mystirinsäure (Tetradecansäure), Pentadecansäure und Palmitinsäure (Hexadecansäure) ausgewählt ist;
• Einheit A und A' unabhängig voneinander aus 1, 2 oder 3 Aminosäuren bestehen, die aus der Gruppe der basischen Aminosäuren oder hydrophoben Aminosäuren ausgewählt sind;
• Einheit B aus 1, 2 oder 3 Aminosäuren besteht, die aus der Gruppe (a) der hydrophoben Aminosäuren und (b) der Gruppe der Aminosäuren, die Wasserstoffbrückenbindungen ausbilden, ausgewählt sind;
wobei:
(i) die hydrophoben Aminosäuren ausgewählt sind aus: Ala, Phe, Ile, Leu, Pro, Tyr, Trp, Val, Met, Cys;
(ii) die basischen Aminosäuren ausgewählt sind aus: Lys, His, Arg;
(iii) die Aminosäuren, die Wasserstoffbrückenbindungen ausbilden, ausgewählt sind aus: Asn, Gln, Ser, Thr;
• -NH2 eine C-terminale Amidierung repräsentiert;
wobei A-B-A' eine Sequenz ist, die aus der Gruppe der folgenden Sequenzen ausgewählt ist:
SEQ ID Nr.: 1, SEQ ID Nr.: 2, SEQ ID Nr.: 3, SEQ ID Nr.: 4, SEQ ID Nr.: 5, SEQ ID Nr.: 6, SEQ ID Nr.: 7, SEQ ID Nr.: 8, SEQ ID Nr.: 9, SEQ ID Nr.: 10, SEQ ID Nr.: 11.

2. Das Lipopeptid nach Anspruch 1, wobei die Substruktur A-B-A' 2 bis 4 Wechselpunkte zwischen Aminosäuren der Gruppe (a) und Gruppe (b) oder umgekehrt enthält.

3. Das Lipopeptid nach Anspruch 1 oder 2, wobei mindestens ein Drittel der Aminosäuren aus der Gruppe der proteinogenen, nicht-proteinogenen oder nicht-Standard-Aminosäuren ausgewählt ist.

4. Das Lipopeptid nach Anspruch 1, 2 oder 3, wobei mindestens eine Aminosäure aus der Gruppe der D-Aminosäuren ausgewählt ist.

5. Das Lipopeptid nach einem der Ansprüche 1 bis 4, wobei FA als Cn definiert ist, wobei n eine ganze Zahl ist, die aus 12, 14, 15, 16 ausgewählt ist, sodass Cn eine Fettsäure repräsentiert, die aus Laurinsäure (Dodecansäure), Mystirensäure (Tetradecansäure), Pentadecansäure und Palmitinsäure (Hexadecansäure) ausgewählt ist.

6. Das Lipopeptid nach Anspruch 1, wobei n 12 ist, sodass FA Lauryl ist.

7. Eine Zusammensetzung umfassend mindestens ein Lipopeptid nach den vorhergehenden Ansprüchen und einen pharmazeutisch akzeptablen Träger.

8. Die Zusammensetzung nach Anspruch 7, wobei das Lipopeptid in einer Konzentration im Bereich von etwa 1 µg/mL bis etwa 12,5% (w/v) vorhanden ist.

9. Die Zusammensetzung nach Anspruch 7 oder 8, wobei die Zusammensetzung in Form eines Aerosols, kosmetischen Zubereitung, Sprays, Emulsion, Flüssigkeit, Lotion, Creme, Paste, Salbe, Pulvers oder Schaums vorliegt.

10. Die Zusammensetzung nach Anspruch 9, zur Verwendung bei der Prävention oder Behandlung von Hautinfektionen, die durch Bakterien oder Pilze verursacht werden, Ohrinfektionen, die durch Bakterien oder Pilze verursacht werden, bakterielle oder Pilzvaginose.

11. Die Zusammensetzung nach Anspruch 9, zur Verwendung bei der Prävention oder Behandlung von Infektionen von pflanzlichen Organismen.

12. Die Zusammensetzung nach einem der Ansprüche 9 bis 11, zur Verwendung bei der Prävention oder Behandlung einer mikrobiellen Infektion der Haut oder des Schleimhautgewebes eines Säugers durch Verabreichung einer therapeutisch wirksamen Menge der Zusammensetzung nach einem der Ansprüche 9 bis 11 an den infizierten Bereich des Säugers.

13. Die Zusammensetzung zur Verwendung nach Anspruch 12, wobei die therapeutisch wirksame Menge der Zusammensetzung das Lipopeptid in einer Konzentration im Bereich von etwa 1 µg/mL bis etwa 12,5% (w/v) umfasst.

## Revendications

1. Lipopeptide ayant une séquence FA-A-B-A'-NH2, dans lequel :
• FA représente un acide gras choisi parmi l'acide caproïque (hexanoïque), l'acide caprylique (octanoïque), l'acide caprique (décanoïque), l'acide laurique (dodécanoïque), l'acide mystirique (tétradécanoïque), l'acide pentadécanoïque et l'acide palmitique (hexadécanoïque) ;
• les unités A et A' sont constituées indépendamment de 1, 2 ou 3 acides aminés choisis dans le groupe constitué d'acides aminés basiques ou d'acides aminés hydrophobes ;
• l'unité B est constituée de 1, 2 ou 3 acides aminés, choisis dans le groupe constitué (a) d'acides aminés hydrophobes et (b) du groupe d'acides aminés formant une liaison hydrogène ;
dans lequel :
(i) lesdits acides aminés hydrophobes sont choisis parmi : Ala, Phe, Ile, Leu, Pro, Tyr, Trp, Val, Met, Cys ;
(ii) lesdits acides aminés basiques sont choisis parmi : Lys, His, Arg ;
(iii) lesdits acides aminés formant une liaison hydrogène sont choisis parmi Asn, Gin, Ser, Thr ;
• -NH2 représente une amidation C-terminale ;
dans lequel A-B-A' est une séquence choisie dans le groupe constitué des séquences suivantes : SEQ ID No : 1, SEQ ID No : 2, SEQ ID No : 3, SEQ ID No : 4, SEQ ID No : 5, SEQ ID No : 6, SEQ ID No : 7, SEQ ID No : 8, SEQ ID No : 9, SEQ ID No : 10, SEQ ID No : 11.

2. Lipopeptide selon la revendication 1, dans lequel la sous-structure A-B-A' contient de 2 à 4 points d'alternance entre des acides aminés du groupe (a) et du groupe (b), ou vice versa.

3. Lipopeptide selon la revendication 1 ou 2, dans lequel au moins un tiers desdits acides aminés sont choisis dans le groupe constitué d'acides aminés protéinogènes, non protéinogènes ou non standard.

4. Lipopeptide selon la revendication 1, 2 ou 3, dans lequel au moins un acide aminé est choisi dans le groupe constitué d'acides D-aminés.

5. Lipopeptide selon l'une quelconque des revendications 1 à 4, dans lequel FA est défini comme Cn, dans lequel n est un entier choisi parmi 12, 14, 15, 16 de telle sorte que Cn représente un acide gras choisi parmi : l'acide laurique (dodécanoïque), l'acide mystirique (tétradécanoïque), l'acide pentadécanoïque et l'acide palmitique (hexadécanoïque).

6. Lipopeptide selon la revendication 1, dans lequel n vaut 12, de telle sorte que FA est Lauryle.

7. Composition comprenant au moins un lipopeptide selon l'une quelconque des revendications précédentes et un support pharmaceutiquement acceptable.

8. Composition selon la revendication 7, dans laquelle ledit lipopeptide est présent à une concentration allant d'environ 1 µg/mL à environ 12,5 % (p/v).

9. Composition selon les revendications 7 ou 8, dans laquelle ladite composition se présente sous forme d'aérosol, de préparation cosmétique, de spray, d'émulsion, de liquide, de lotion, de crème, de pâte, de pommade, de poudre ou de mousse.

10. Composition selon la revendication 9 à utiliser dans la prévention ou le traitement d'infections cutanées causées par des bactéries ou des champignons, des infections de l'oreille causées par des bactéries ou des champignons, des vaginoses bactériennes ou fongiques.

11. Composition de la revendication 9 à utiliser dans la prévention ou le traitement d'infections d'organismes végétaux.

12. Composition selon l'une quelconque des revendications 9 à 11 à utiliser dans la prévention ou le traitement d'une infection microbienne de la peau ou d'un tissu muqueux d'un mammifère par administration à la zone infectée dudit mammifère d'une quantité thérapeutiquement efficace de ladite composition selon l'une quelconque des revendications 9 à 11.

13. Composition à utiliser selon la revendication 12, dans laquelle ladite quantité thérapeutiquement efficace de la composition comprend ledit polipeptide à une concentration allant d'environ 1 µg/mL à environ 12,5 % (p/v).
